**Europäisches Patentamt**

**European Patent Office**

**Office européen des brevets**

(19)

(11) Veröffentlichungsnummer : **0 309 441 B1**

(12)

# EUROPÄISCHE PATENTSCHRIFT

(45) Veröffentlichungstag der Patentschrift :
**29.04.92 Patentblatt 92/18**

(51) Int. Cl.⁵ : **A61F 2/50,** F15B 15/14

(21) Anmeldenummer : **88890241.8**

(22) Anmeldetag : **21.09.88**

(54) Doppelt wirkende hydraulische Kolben-Zylinder-Einheit.

(30) Priorität : **22.09.87 AT 2399/87**

(43) Veröffentlichungstag der Anmeldung :
**29.03.89 Patentblatt 89/13**

(45) Bekanntmachung des Hinweises auf die
Patenterteilung :
**29.04.92 Patentblatt 92/18**

(84) Benannte Vertragsstaaten :
**AT BE CH DE ES FR GB IT LI LU NL SE**

(56) Entgegenhaltungen :
**EP-A- 0 097 226**
**AT-B- 379 000**
**DE-B- 1 491 236**

(73) Patentinhaber : **Otto Bock Orthopädische
Industrie Besitz- und Verwaltungs-KG
Industriestrasse
W-3428 Duderstadt (DE)**

(72) Erfinder : **Horvath, Eduard, Ing.
Kaiserstrasse 39
A-1070 Wien (AT)**

(74) Vertreter : **Casati, Wilhelm, Dipl.-Ing. et al
Patentanwälte Casati, Wilhelm, Dipl.-Ing. Itze,
Peter, Dipl.-Ing. Amerlingstrasse 8
A-1061 Wien (AT)**

## Beschreibung

Die Erfindung bezieht sich auf eine doppelt wirkende hydraulische Kolben-Zylinder-Einheit, insbesondere für die Regelung der Bewegung künstlicher Gelenke an Menschen, bei welcher der Zylinder mit einem elastisch dehnbaren, zur Aufnahme der aus dem Zylinderinnenraum verdrängten, unter Druck stehenden Flüssigkeit dienenden Balg umgeben ist, der einen zylindermantelförmigen Abschnitt und einen anschließenden Abschnitt, der mit dem zylindermantelförmigen Abschnitt verbunden ist, aufweist, wobei der Innenraum des Balges mit dem Zylinderinnenraum kommuniziert, und wobei der Balg mit seinem zylindrischen Abschnitt am Zylinder und mit seinem anschließenden Abschnitt an der Kolbenstange dicht befestigt ist.

Bei einer bekannten Ausbildung dieser Art schließt der sich erweiternde Abschnitt an den zylindermantelförmigen Abschnitt des Balges direkt an, wobei dieser Anschlußbereich im Bereich des oberen Endes des Zylinders angeordnet ist. Der von der Kolbenstange ausgehende, sich erweiternde Bereich geht dabei unter relativ spitzem Winkel von der Kolbenstange weg, so daß der Übergangsbereich zwischen zylindrischem Abschnitt und sich erweiterndem Abschnitt des Balges nur schwach ausgebildet ist. Außerdem erstreckt sich der sich erweiternde Bereich über die gesamte Länge des Kolbenhubes, so daß der Winkel, unter welchem der sich erweiternde Abschnitt von der Kolbenstange wegführt, sehr spitz ist. Schließlich ist bei der bekannten Ausbildung nahezu das gesamte Volumen jenes Raumes innerhalb des Balges, welches in dem sich erweiternden Bereich liegt, mit Gas gefüllt, da dieser Gaspolster als Federung eingesetzt wird. Eine solche Ausbildung hat den Nachteil, daß beim Einschieben der Kolbenstange in den Zylinder der sich erweiternde Abschnitt des Balges in Form einer Rollmembran zusammenschiebt, wobei das beim Zusammenschieben verringerte Volumen des Balginnenraumes durch eine radiale Dehnung des Balges kompensiert wird. Diese radiale Dehnung ist aber zufolge des nicht ausgeprägten überganges zwischen den beiden Abschnitten des Balges und der Ausbildung als Rollmembran sowie durch das Vorhandensein des Gaspolsters innerhalb des Balges undefiniert, d.h., daß sich der Balg in einer Weise verformen kann, daß seine Außenkontur von einem Zylindermantel weit abweicht. Dies ist aber insbesondere bei künstlichen Gelenken an Menschen nachteilig, da dort der Platz sehr beschränkt ist, wobei dann unkontrollierte Ausbauchungen der Membran und gegebenenfalls ein Scheuern derselben durch die Bewegung zu Defekten der Dämpfungseinrichtung führen kann.

Der Erfindung liegt die Aufgabe zugrunde, eine doppelt wirkende hydraulische Kolben-Zylinder-Einheit der eingangs genannten Art zu schaffen, bei welcher die Außenabmessungen des Balges eine etwa vorgegebene Form beibehalten sollen.

Erfindungsgemäß wird diese Aufgabe dadurch gelöst, daß der übergang zwischen dem zylindrischen Abschnitt und dem von der Kolbenstange ausgehenden, anschließenden Abschnitt des Balges durch einen, vorzugsweise wulstartigen, Bereich gebildet ist, der größeren Durchmesser als der zylindrische Abschnitt aufweist, und daß alle hydraulisch wirksamen Hohlräume, also der Innraum des Balges und die Zylinderräume zu beiden Seiten des Kolbens, mit Hydraulikflüssigkeit gefüllt sind, und wobei das Verhältnis zwischen dem Radius des Bereiches, der größeren Durchmesser als der zylindrische Abschnitt aufweist, und dem axialen Abstand zwischen der Mittelebene der Bereichs, der größeren Durchmesser als der zylindrische Abschnitt aufweist, und der Befestigung des Balges an der Kolbenstange zwischen 3 : 1 bis 1 : 2, vorzugsweise 2 : 1, beträgt. Dadurch wird erreicht, daß zufolge des sich erweiternden Bereiches im übergang zwischen dem zylindrischen Abschnitt und dem sich erweiternden Abschnitt des Balges und des Verhältnisses zwischen dem Radius des Bereiches, der größeren Durchmesser als der zylindrische Abschnitt aufweist, und dem axialen Abstand zwischen dem Bereich, der größeren Durchmesser als der zylindrische Abschnitt aufweist, und der Befestigung des Balges an der Kolbenstange, der von der Kolbenstange ausgehende, sich erweiternde Bereich zunächst als Tellermembran wirkt, was bedeutet, daß im Anfang der Kolbeneinschubbewegung diese Membran eine geringere Volumsverdrängung aufweist als eine analoge Rollmembran, wobei zufolge des Bereiches, der größeren Durchmesser als der zylindrische Abschnitt aufweist, eine Stabilisierung des zylindrischen Bereiches des Balges erfolgt. Es wird dabei bei einer radialen Ausdehnung des Balges zunächst der zwischen der Befestigung des freien Endes des zylindrischen Abschnittes am Zylinder und dem Bereich, der größeren Durchmesser als der zylindrische Abschnitt aufweist, liegende Teil des zylindrischen Abschnittes erweitert, so daß der gesamte zylindrische Bereich über seine von der Anbringung bis zum Ende des Bereiches, der größeren Durchmesser als der zylindrische Abschnitt aufweist, liegenden Teil Zylinderform aufweist. Erst wenn dann der Kolben weiter hineingedrückt wird, geht der zunächst als Tellermembran wirkende, sich erweiternde Abschnitt in eine Rollmembran über.

Vorteilhafterweise kann in dem von der Kolbenstange ausgehenden, sich erweiternden Abschnitt des Balges, vorzugsweise an den Bereich, der größeren Durchmesser als der zylindrische Abschnitt aufweist, anschließend, ein Bereich, der größere Flexibilität als der übrige Balg aufweist, vorgesehen sein, wodurch die Bewegung der Membran während des überganges von der Tellermembran in die Rollmembran genau gesteuert werden kann. Schließlich kann der zylindrische Abschnitt des Balges im Bereich des Anschlusses an den Zylinder einen, vorzugsweise ebenfalls wulstartigen, Bereich aufweisen, der größeren Durchmesser als der zylindrische Abschnitt besitzt, was gleichfalls dazu beiträgt, daß der Balg im voll eingefahrenen Zustand der Kolbenstange etwa zylindrische Außenkontur aufweist.

In der Zeichnung ist ein Ausführungsbeispiel des Erfindungsgegenstandes dargestellt.

Fig. 1 zeigt einen Axialschnitt durch den Erfindungsgegenstand bei ausgefahrener Kolbenstange.

Fig. 2 gibt einen analogen Schnitt, jedoch bei eingefahrener Kolbenstange, wieder.

Mit 1 ist ein Hydraulikzylinder bezeichnet, in welchem ein mit einer Kolbenstange 2 versehener Kolben 3 in axialer Richtung verschiebbar geführt ist. Der Hydraulikzylinder ist dabei an seinem der Kolbenstange abgewandten Ende durch einen Zylinderboden 4 und an seinem die Kolbenstange aufweisenden Ende durch einen Zylinderdeckel 5 abgeschlossen, durch welchen die Kolbenstange dicht hindurchgeführt ist. Außen ist der Zylinder 1 und die Kolbenstange 2 durch einen flexiblen, elastisch dehnbaren Balg 6 abgeschlossen, welcher mit seinem einen Ende am Zylinder und mit seinem anderen Ende an der Kolbenstange dicht verbunden ist.

Der Balg 6 weist dabei einen etwa zylindrischen Abschnitt 7 und einen von der Kolbenstange ausgehenden, sich erweiternden Abschnitt 8 auf. Im Bereich des Überganges zwischen dem zylindrischen Teil 7 und dem sich erweiternden Teil 8 ist ein Bereich 9, der größeren Durchmesser als der zylindrische Abschnitt aufweist, vorgesehen, der vorliegend als Wulst ausgebildet ist. An dem vom Wulst 9 abgewandten Ende ist der zylindrische Abschnitt 7 das Balges 6 mit einem Wulst 10 versehen, welcher zur Befestigung des Balges am Zylinder 1 hinführt. Der sich erweiternde Abschnitt 8 weist dabei eine Konizität auf, bei welcher der Radius R des Bereiches 9 der größeren durchmesser als der zylindrische Abschnitt aufweist etwa der doppelten Entfernung L der Mittelebene E des Bereiches 9, der größeren Durchmesser als der zylindrische Abschnitt aufweist, von der Anbringung 11 des Balges 6 an der Kolbenstange entspricht. An den Bereich größeren Durchmessers 9 schließt in dem sich erweiternen Abschnitt 8 des Balges 6 ein Bereich größerer Flexibilität 12 an, welcher der Funktion des sich erweiternden Abschnittes als Tellermembran zu Beginn der Bewegung zuträglich ist.

Der Innenraum des Balges 6 ist mit dem Zylinderinnenraum 13 unterhalb des Kolbens 3 über eine Verbindungsleitung 14 verbunden, in welcher ein Rückschlagventil 15 vorgesehen ist, welches einen Durchtritt der Hydraulikflüssigkeit vom Zylinderraum 13 in den Innenraum des Balges verhindert. Weiters ist der Zylinderraum 13 mit dem Innenraum des Balges 6 über eine Drossel 16 verbunden. Der Zylinderraum 17 oberhalb des Kolbens 3 ist mit dem Innenraum des Balges 6 über eine Verbindungsleitung 18 verbunden, in welcher ein Rückschlagventil 19 eingebaut ist, welches den Durchfluß der Flüssigkeit vom Zylinderraum 17 zum Innenraum des Balges 6 verhindert. Weiters steht der Zylinderraum 17 mit dem Innenraum des Balges 6 noch über eine Drossel 20 in Verbindung.

21 sind Bohrungen für die Befestigung des Zylinderbodens an einer Tragplatte, und 22 eine Bohrung zur Anlenkung der Kolbenstange an ein Betätigungsorgan.

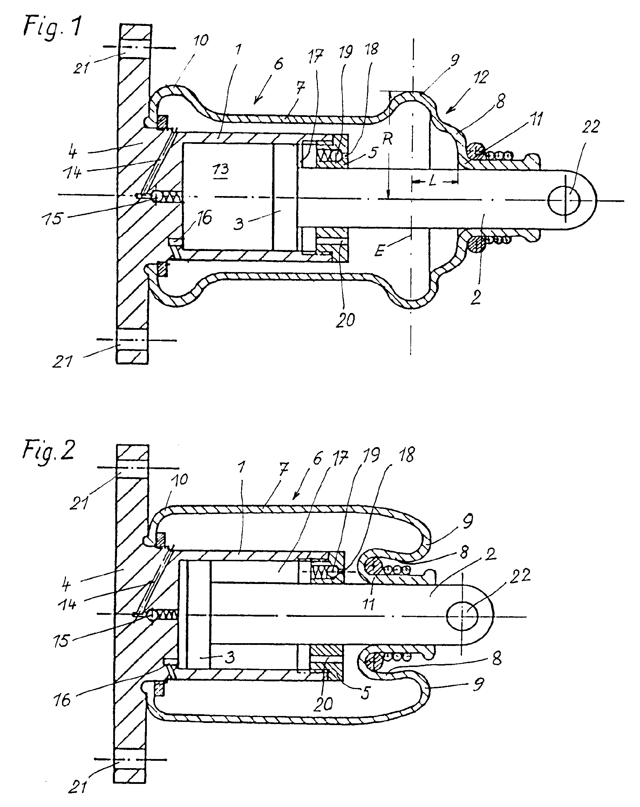

Wird die Kolbenstange 2 von der in Fig. 1 gezeigten Stellung ausgehend, in den Zylinder eingefahren, dann wird die im Zylinderraum 13 unterhalb des Kolbens 3 befindliche Hydraulikflüssigkeit durch die Drossel 16 in den Innenraum des Balges 6 gepreßt, wobei durch den sich im Zylinderraum 17 oberhalb des Kolbens bildenden Unterdruck Hydraulikflüssigkeit aus dem Innenraum des Balges einerseits über die Drossel 20 und andererseits über die Verbindungsleitung 18 eingesaugt wird. Da das Volumen der aus dem Zylinderraum 13 hinausgeförderten Hydraulikflüssigkeit größer ist als das in den Zylinderraum 17 eingesaugte Flüssigkeitsvolumen, und andererseits zufolge der Verkürzung des Abstandes L aufgrund der Einwärtsbewegung der Kolbenstange das Volumen des im Bereich des sich erweiternden Abschnittes befindlichen Teiles des Balges verringert, wird der zylindrische Bereich 7 des Balges 6 radial erweitert, und zwar so weit, bis die in Fig. 2 wiedergegebene Stellung der Teile erreicht ist. Während dieser Bewegung wird sich der sich erweiternde Abschnitt 8 des Balges 6 im ersten Teil der Bewegung wie eine Scheibenmembran verhalten, und zwar dahingehend, daß sich dieser besagte Abschnitt 8 zunächst verflacht, also in eine nahezu ebene Stellung übergeht, und dann nach innen stülpt, und zwar solange, bis der Bereich der Befestigung 11 des Balges 6 an der Kolbenstange 2 innerhalb des Bereiches 9 größeren Durchmessers zu liegen kommt, wodurch es also zu einer Einstülpung des Abschnittes 8 des Balges 6 kommt. Diese Einstülpung wird dabei solange vorgenommen, bis sich auch der Bereich 9 nach innen rollt. Die gegenseitige Lage der einzelnen Teile in dieser Endstellung des Kolbens ist in Fig. 2 wiedergegeben.

Wird nun der Kolben 3 und damit die Kolbenstange aus dem Zylinder ausgefahren, dann tritt der gegenteilige Bewegungsablauf und Flüssigkeitsstrom auf, nämlich dahingehend, daß Hydraulikflüssigkeit aus dem Innenraum des Balges 6 durch die Verbindungsleitung 14 über das Rückschlagventil 15 und auch teilweise über die Drossel 16 in den Zylinderraum 13 unterhalb des Kolbens 3 eingesaugt wird. Gleichzeitig wird jene Hydraulikflüssigkeit, die sich im Zylinderraum 17 oberhalb des Kolbens befindet, über die Drossel 20 in den Innenraum des Balges 6 gedrängt. Da das eingesaugte Volumen größer als das verdrängte Volumen ist und andererseits aufgrund der Streckung des Balges das Volumen des Balginnenraumes vergrößert wird, verringert sich der Durchmesser des zylindrischen Teiles des Balges, wobei zufolge der beidseitigen Begrenzung des

3

zylindrischen Bereiches 7 des Balges 6 durch die wulstartigen Erweiterungen 9, 10 ein gleichmäßiges Einziehen des Balges erzielt wird. Am Ende der Ausfahrbewegung der Kolbenstange 2 nehmen die einzelnen Teile der Einrichtung wieder die in Fig. 1 wiedergegebene Lage ein.

**Patentansprüche**

1. Doppelt wirkende hydraulische Kolben-Zylinder-Einheit, insbesondere für die Regelung der Bewegung künstlicher Gelenke an Menschen, bei welcher der Zylinder (1) mit einem elastisch dehnbaren, zur Aufnahme der aus dem Zylinderinnenraum verdrängten, unter Druck stehenden Flüssigkeit dienenden Balg (6) umgeben ist, der einen zylindermantelförmigen Abschnitt (7) und einen anschließenden Abschnitt (8), der mit dem zylindermantelförmigen Abschnitt (7) verbunden ist, aufweist, wobei der Innenraum des Balges (6) mit dem Zylinderinnenraum kommuniziert und wobei der Balg (6) mit seinem zylindrischen Abschnitt (7) am Zylinder (1) und mit seinem anschließenden Abschnitt (8) an der Kolbenstange (2) dicht befestigt ist, dadurch gekennzeichnet, daß der Übergang zwischen dem zylindrischen Abschnitt (7) und dem von der Kolbenstange (2) ausgehenden, anschießenden Abschnitt (8) des Balges (6) durch einen, vorzugsweise wulstartigen, Bereich (9) gebildet ist, der größeren Durchmesser als der zylindrische Abschnitt (7) aufweist, und daß alle hydraulisch wirksamen Hohlräume, also der Innenraum des Balges (6) und die Zylinderräume (13, 17) zu beiden Seiten des Kolbens (3) mit Hydraulikflüssigkeit gefüllt sind, und wobei das Verhältnis zwischen dem Radius (R) des Bereiches (9) und dem axialen Abstand (4) zwischen der Mittelebene (E) des Bereichs (9) und der Befestigung (11) des Balges (6) an der Kolbenstange (2) zwischen 3 : 1 bis 1 : 2, vorzugsweise 2 : 1, beträgt.

2. Kolben-Zylinder-Einheit nach Anspruch 1, dadurch gekennzeichnet, daß in dem von der Kolbenstange (2) ausgehenden, sich erweiternden Abschnitt (8) des Balges (6), vorzugsweise an den Bereich (9) anschließend, ein Bereich (12), der größere Flexibilität als der übrige Balg aufweist, vorgesehen ist.

3. Kolben-Zylinder-Einheit nach Anspruch 1 oder 2, dadurch gekennzeichnet, daß der zylindrische Abschnitt (7) des Balges (6) im Bereich des Anschlusses an den Zylinder (1) einen, vorzuqsweise ebenfalls wulstartigen, Bereich (10) aufweist, der größeren Durchmesser als der zylindrische Bereich (7) besitzt.

**Claims**

1. Double-acting hydraulic piston-cylinder unit, in particular for controlling the movement of artificial joints in humans, in which the cylinder (1) is surrounded by an elastically expandable bellows (6) which serves to receive the pressurised fluid displaced from the interior of the cylinder and has a cylinder envelope-shaped portion (7) and an adjoining portion (8) which is connected to the cylinder envelope-shaped portion (7), the interior of the bellows (6) communicating with the interior of the cylinder and the bellows (6) being secured in leakproof fashion on the cylinder (1) with its cylindrical portion (7) and in leakproof fashion on the piston rod (2) with its adjoining portion (8), characterised in that the transition between the cylindrical portion (7) and the adjoining portion (8) of the bellows (6), which portion (8) starts from the piston rod (2), is formed by a preferably bead-like region (9) which has a larger diameter than the cylindrical portion (7) and in that all the hydraulically acting cavities, i.e. the interior of the bellows (6) and the cylinder chambers (13, 17) on the two sides of the piston (3) are filled with hydraulic fluid, and in this arrangement the ratio between the radius (R) of the region (9) and the axial distance (4) between the centre plane (E) of region (9) and the fixing (11) of the bellows (6) on the piston rod (2) is between 3 : 1 to 1 : 2, preferably 2 : 1.

2. Piston-cylinder unit according to Claim 1, characterised in that a region (12) which has greater flexibility than the remainder of the bellows and preferably adjoins region (9) is provided in the widening portion (8) of the bellows (6), which region starts from the piston rod (2).

3. Piston-cylinder unit according to Claim 1 or 2, characterised in that, in the region of the connection to the cylinder (1), the cylindrical portion (7) of the bellows (6) has a region (10), preferably likewise bead-like, which has a larger diameter than the cylindrical region (7).

**Revendications**

1. Unité hydraulique avec cylindre et piston à double effet, destinée en particulier au réglage du mouvement d'articulations artificielles sur des personnes, dans laquelle le cylindre (1) est entouré d'un soufflet (6) élastiquement extensible, servant à recueillir le liquide sous pression refoulé de l'intérieur du cylindre, présentant une portion (7) en forme de corps de cylindre et une portion lui faisant suite (8), reliée à la portion en forme de corps de cylindre (7), l'intérieur du soufflet (6) communiquant avec l'intérieur du cylindre, et le soufflet (6) étant fixé de façon étanche, par sa portion cylindrique (7) au cylindre (1), et par sa portion lui faisant suite (8) à la tige de piston (2), caractérisée en ce que la transition entre la portion cylindrique (7) et la portion (8) du soufflet (6) s'étendant à partir de la tige de piston (2) et faisant suite à la portion (7) est formée par une zone (9) de préférence en forme de bourrelet, présentant un diamètre supérieur à celui de la portion cylindrique (7), et en ce que toutes les cavités hydrauliquement actives, donc l'intérieur du soufflet (6) et les chambres de cylindre (13, 17), sont remplies de fluide hydraulique des deux côtés du piston (3), le rapport entre le rayon (R) de la zone (9) et l'écart axial (4) séparant le plan médian (E) de la zone (9) et la fixation (11) du soufflet (6) sur la tige de piston (2) se situant de préférence entre 3 : 1 et 1 : 2, et étant de préférence de 2 : 1.

2. Unité cylindre-piston selon la revendication 1, caractérisée en ce qu'est prévue, dans la portion (8) du soufflet (6) s'étendant à partir de la tige de piston (2) et s'élargissant, et de préférence adjacente à la zone (9), une zone (12) présentant une plus grande flexibilité que le reste du soufflet.

3. Unité cylindre-piston selon la revendication 1 ou 2, caractérisée en ce que la portion cylindrique (7) du soufflet (6) présente, dans la zone de raccordement au cylindre (1), une zone (10) de préférence également en forme de bourrelet, présentant un diamètre supérieur à celui de la portion cylindrique (7).

Fig. 1

Fig. 2